(19) Europäisches Patentamt
European Patent Office
Office européen des brevets

(11) Publication number: **0 054 384**
**B1**

(12) **EUROPEAN PATENT SPECIFICATION**

(45) Date of publication of patent specification: **27.06.84**

(21) Application number: **81305724.7**

(22) Date of filing: **04.12.81**

(51) Int. Cl.[3]: **C 07 D 215/48,**
C 07 D 219/04,
C 07 D 221/04,
C 07 D 221/12,
C 07 D 221/16 //A61K31/47,
A61K31/435

(54) **Process for preparing pyridine derivatives.**

(30) Priority: **09.12.80 GB 8039423**
**23.11.81 GB 8135212**

(43) Date of publication of application:
**23.06.82 Bulletin 82/25**

(45) Publication of the grant of the patent:
**27.06.84 Bulletin 84/26**

(84) Designated Contracting States:
**AT BE CH DE FR IT LI LU NL SE**

(56) References cited:
**GB - A - 1 432 378**
**GB - A - 1 444 205**
**GB - A - 1 453 665**
**GB - A - 1 458 148**
**GB - A - 1 463 666**
**GB - A - 1 463 667**
**GB - A - 1 463 668**
**GB - A - 1 463 669**
**GB - A - 1 471 371**
**GB - A - 1 495 993**

(73) Proprietor: **JOHN WYETH & BROTHER LIMITED**
**Huntercombe Lane South Taplow**
**Maidenhead Berkshire, SL6 0PH (GB)**

(72) Inventor: **Shepherd, Robin Gerald**
**79, Huntercombe Lane North**
**Burnham Buckinghamshire (GB)**

(74) Representative: **Porter, Graham Ronald et al,**
**C/O John Wyeth & Brother Limited Huntercombe Lane South Taplow**
**Maidenhead Berkshire, SL6 0PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 054 384 B1**

**Description**

The invention relates to a process for preparing pyridine derivatives which have value as anti-ulcer/anti-secretory agents.

UK Patent Specification 1,432,378 describes fused carbocyclic ring derivatives of pyridine, for example 8-cyano-5,6,7,8-tetrahydroquinolines and the corresponding thioamides which are either anti-ulcer/anti-secretory agents or intermediates for such agents.

Analogous nitriles and thioamides are described in UK Patent Specifications 1,495,993 and 1,458,148. Further examples of such nitriles and thioamides are given in UK Specifications 1,463,668; 1,463,669 and 1,471,371.

These specifications describe various ways of making the nitriles and thioamides. However, it has been found that the previously described methods do not always give high yields of the desired end product. We have now found a new method for preparing the nitriles and thioamides which is often superior to the previous routes.

According to the present invention there is provided a process for preparing compounds of Formula I

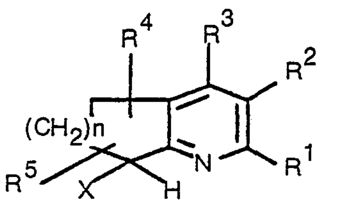

(I)

or acid addition salts thereof, wherein $R^1$, $R^2$, $R^3$, $R^4$ and $R^5$ are the same or different and represent hydrogen alkyl, cycloalkyl, aralkyl, or aryl radicals, any of which radicals may be substituted or $R^1$ and $R^2$ taken together, or $R^2$ and $R^3$ taken together, form a 5,6, or 7 membered ring which may be saturated or unsaturated and substituted or unsubstituted, $R^4$ and $R^5$ may also represent alkoxy, n is 1, 2 or 3 and X is CN or CSNHR characterised in that a compound of Formula II

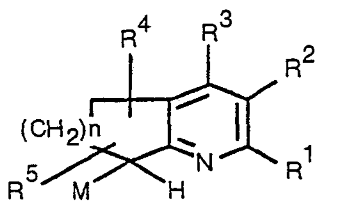

(II)

wherein $R^1$, $R^2$, $R^3$, $R^4$, $R^5$ and n are as defined above, and M is lithium, sodium, potassium, or MgHal, where Hal is chlorine, bromine or iodine, is reacted with a compound of Formula RaRbNCN wherein Ra and Rb are the same or different and represent alkyl, cycloalkyl or aralkyl, or Ra and Rb may be joined to form a heterocyclic ring with the nitrogen atom to which they are attached and the product is treated with a proton source to obtain a nitrile of Formula I wherein X is CN and if desired the nitrile is treated with a sulphurising agent to obtain a thioamide of Formula I wherein X is CSNHR where R is hydrogen or lower alkyl and optionally the compound of Formula I is isolated as an acid addition salt.

The starting compounds of Formula II may be prepared as discussed in any of the aforementioned prior UK Patent Specifications, or in UK Patent Specifications 1463665 and 1463666. The preferred method for introducing the metal ion M is as described in UK Patent 1463666 namely by reaction of a corresponding compound of Formula II wherein M is hydrogen with a metal alkyl $MR^6$ wherein M is sodium, potassium or lithium and $R^6$ is alkyl, (e.g. n-butyl), aralkyl or aryl e.g. phenyl or mesityl (2,4,6-trimethyphenyl) or with a metal amide MA wherein M is sodium, potassium or lithium and A is an amine radical, preferably a secondary amine.

The metal amide may be formed *in situ* by reacting a metal compound $MR^6$ as defined above with an amine $R^9R^{10}NH$, wherein $R^9$ and $R^{10}$ have the same meanings as Ra and Rb, or $R^9$ is hydrogen, preferably in a molar amount equal to that of the metal alkyl. The amine is preferably a secondary amine such as a dialkylamine, e.g. diethylamine, di-isopropylamine, di-t-butylamine, di-n-decylamine, dicyclohexylamine, N-t-amyl-N-t-butylamine, N-isopropyl-N-cyclohexylamine, N-t-butyl-N-cyclohexylamine, N-t-butyl-$\alpha$-methylbenzylamine or N(1-ethylcyclohexyl)-1,1,3,3-tetramethylbutylamine or a cyclic amine e.g. piperidine, or an alkyl substituted piperidine e.g. 2,2,6,6-tetramethylpiperidine. Preferred amines are hindered amines such as one in which $R^9$ and $R^{10}$ are selected from secondary or tertiary radicals or cycloalkyl radicals, especially when $R^3$ is alkyl.

When forming the starting compound of Formula II from a corresponding compound in which M is hydrogen the yield may be reduced by competing reactions when $R^1$ or $R^3$ contain exchangeable hydrogen atoms, e.g. when $R^1$ or $R^3$ are alkyl. In such cases a mixture may be formed with the desired compound of Formula II and one in which M is hydrogen and there is a metal substituent on $R^1$ or $R^3$.

The reaction with RaRbNCN then produces as impurity a compound with the CN group in the radical $R^1$ or $R^3$. The mixture of desired product of Formula I and impurity may be separated by conventional methods, e.g. crystallisation, chromatography, distillation and so on. When competing metallations are liable to occur, it is possible to favour formation of compound II by appropriate choice of reaction conditions and/or reagents. For example when $R^3$ is alkyl it has been found that formation of the compound of Formula II is favoured or exclusive when the starting compound wherein M is hydrogen is added to a solution of a small excess of the metal alkyl or up to 2.5 molar equivalents of the metalamide, rather than the converse.

When an alkyl group $R^3$ is present in compound II it has been found that the reaction with compound RaRbNCN may still produce some of the "wrong" isomer. The amount of "wrong" isomer can be considerably reduced by adding compound II to a refluxing solution of RaRbNCN instead of the reverse of this procedure. Yields are even higher when metal amide (preferably a molar equivalent of metal amide) is present during the reaction with compound RaRbNCN. If the starting compound II is formed *in situ* using 2 molar equivalents of metal amide this provides the excess metal amide for use during the reaction with RaRbNCN. It has also been found that when the metal amide is of a hindered secondary amine, e.g. N-t-butyl-N-cyclohexylamine, formation of the desired isomer is favoured.

The process of the invention is carried out by reacting compound II with RaRbNCN in a suitable solvent e.g. a hydrocarbon solvent such as benzene, toluene or xylene or an ether solvent e.g. ether, tetrahydrofuran or dioxan. The temperature may range from —70°C. to the reflux temperature of the solvent e.g. —20°C. to 80°C. A mixture of solvents may be used e.g. the compound II may be prepared *in situ* in a different solvent from that in which the reaction with RaRbNCN takes place e.g. hexane.

The compound RaRbNCN may be added to a refluxing solution of compound II or vice versa but it is preferred to add compound II to refluxing RaRbNCN when $R^3$ is lower alkyl in compound II. It is also preferable to keep the reaction time short e.g. up to 3 hours.

When any of $R^1$, $R^2$, $R^3$, $R^4$, $R^5$, $R^6$, Ra, Rb or R is an alkyl radical, a lower alkyl of 1—6 carbon atoms is preferred. This may be straight or branched chain e.g. methyl, ethyl, *n*-and *iso* propyl, *n*, *s*- and t-butyl, pentyl or hexyl.

When any of $R^1$, $R^2$, $R^3$, $R^4$, $R^5$, Ra or Rb is a cycloalkyl radical this preferably has from 4 to 6 carbon atoms.

When any of $R^1$,$R^2$,$R^3$,$R^4$,$R^5$,$R^6$, Ra or Rb is an aralkyl radical this is preferably aryl lower alkyl in which the lower alkyl portion has 1—6 carbon atoms and may be any of the radicals specified above for a lower alkyl radical.

An aryl radical as used herein is preferably phenyl, which may be substituted e.g. by alkyl, alkoxy, fluorine, or trifluoromethyl. The alkyl and alkoxy radicals are preferably lower alkyl as discussed above or lower alkoxy in which the alkyl portion is as described above for a lower alkyl radical.

Aralkyl radicals may be substituted on the aryl portion as discussed above for an aryl radical.

Any of the alkyl radicals mentioned above may be substituted by alkoxy or trifluoromethyl.

The reagent RaRbNCN is preferably diisopropyl cyanamide but other examples are dimethyl cyanamide, diethyl cyanamide, ditert-butyl cyanamide, dicyclohexyl cyanamide, N-t-butyl-N-cyclohexyl cyanamide, 1-cyano-piperidine or 1-cyano 2,2,6,6-tetramethylpiperidine. Preferably Ra and Rb are chosen from secondary and tertiary alkyl radicals or cycloalkyl radicals. The product of reaction of RaRbNCN and Compound II is treated with a proton source such as water, e.g. in the form of an aqueous acid or other aqueous medium, an alkanol, e.g. of 1—6 carbon atoms, or ammonium chloride which can be added as a solid or in solution.

The nitriles of Formula I may be used either as anti-ulcer/anti-secretory agents or converted to the corresponding thioamides by treatment with a sulphurising agent e.g. by treatment with $H_2S$ (optionally in the presence of a lower alkylamine $RNH_2$) or thioamide $R^7CSNH_2$ wherein $R^7$ is an alkyl group in a solvent such as pyridine, a lower alkanol, dioxane, dimethyl formamide, dimethyl sulphoxide, sulpholane. A base such as a trialkylamine may be present. Alternatively the sulphurising agent may be a dithio compound of formula $(R^8)_2$ PSSH wherein $R^8$ is alkyl, aralkyl, aryl, alkoxy, aralkoxy or aryloxy which is used in the presence of a hydrogen halide. A thioamide of Formula I wherein X is $CSNH_2$ may be converted into a thioamide where X is CSNHR by treatment with a lower alkylamine. The reaction may be carried out with or without an inert solvent, suitable solvents for the reaction with the dithio acid include chlorinated hydrocarbons such as dichloromethane, dichloroethane, chloroform, carbon tetrachloride or hydrocarbons such as benzene and toluene. Particularly preferred dithio acids are diethyldithiophosphate and diphenylphosphinodithioic acid.

The nitriles or thioamides may be converted to acid addition salts with inorganic acids e.g. hydrochloric, hydrobromic, sulphuric or nitric acids or organic acids e.g. citric, fumaric, maleic, or tartaric acids or organic sulphonic acids such as alkyl sulphonic acids e.g. methane sulphonic acid or aryl sulphonic acids e.g. p-toluene sulphonic acid.

Preferably the compounds prepared according to the invention have Formula III

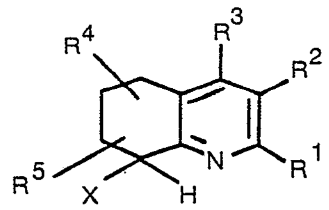

(III)

wherein $R^1$,$R^2$,$R^3$,$R^4$,$R^5$ and X are as defined above.

Especially preferred are those compounds obtained from a starting material of Formula IV

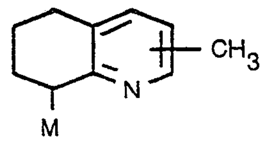

(IV)

wherein M is as defined above.

Example 1

8-Cyano-4-methyl-5,6,7,8-tetrahydroquinoline

A solution of 4-methyl-5,6,7,8-tetrahydroquinoline (73.5 g, 0.5 M) in tetrahydrofuran (300 ml) was added to a 1.6 M solution of butyl lithium in hexane (344 ml, 0.55 M) maintained at 0°C. After 10 min. the resulting red anion solution was added to a refluxing mixture of diisopropyl cyanamide (94.5 g, 0.75 M) in tetrahydrofuran (300 ml). The reaction mixture was cooled and poured on to iced water (500 ml) neutralised with 12 M hydrochloric acid and the organic solvent removed under reduced pressure. The residue was adjusted to pH 1 (12 M HCl) and extracted with ethyl acetate (2 × 500 ml). The aqueous phase was adjusted to pH 10 (potassium carbonate) and extracted with dichloromethane (3 × 300 ml). The final organic extracts were dried and the solvent removed. The residue was dissolved in methanol and treated with an excess of ethereal hydrogen chloride. Removal of the resulting crystals by filtration gave the title compound as the hydrochloride (73 g, 70%) identical with authentic material. mp 253—5°C.

Found: C,63.0, H,6.7; N,13.1, $C_{11}H_{12}N_2$.HCl requires C.63.3; H,6.3; N,13.4%.

Example 2

8-Cyano-5,6,7,8-tetrahydroquinoline

A solution of 5,6,7,8-tetrahydroquinoline (6.5 ml, 50 mM) in benzene (35 ml) maintained below 10° was treated with 1.6 M butyl lithium in hexane (31.25 ml, 50mM). The resulting anion solution was heated to reflux then treated with a solution of diisopropylcyanamide (6.3 g, 50 mM) in benzene (15 ml). After 15 min the mixture was cooled and quenched with water (20 ml). The organic phase was separated, dried and evaporated. Distillation of the residue gave the title compound (5.6 g 71%) Bp. 135—140°/2 mm, identical with authentic material. Hydrochloride mp 185°C — see Example 37 UK Patent 1,432,378.

Example 3

8-Cyano-5,6,7,8-tetrahydroquinoline

A solution of 8-lithio-5,6,7,8-tetrahydroquinoline (10 mM) was generated from 5,6,7,8-tetrahydroquinoline (1.3 ml), ether (6 ml) and 1.6 M butyl lithium in hexane (6.25 ml) then added to a solution of diisopropylcyanamide (3.9 g, 30 mM) in ether (6 ml) at 0°C.

Work up of the reaction mixture after 15 minutes as described in Example 2 gave the title compound in 85% yield.

Example 4

8-Cyano-3-methyl-5,6,7,8-tetrahydroquinoline

Following the procedure of Example 1 3-methyl-5,6,7,8-tetrahydroquinoline is treated with butyllithium in hexane and then the reaction mixture is added to a refluxing solution of diispropyl cyanamide in tetrahydrofuran. The reaction mixture is treated with water and the title compound isolated as the hydrochloride, after the manner of Example 1, mp 189°C.

Example 5

Following the procedure of Example 1 but using alternative starting materials, the following compounds are prepared.

| Starting Material | RaRbNCN | Product |
|---|---|---|
| a) 2-Phenyl-5,6,7,8-tetrahydroquinoline | diisopropylcyanamide | 8-cyano-2-phenyl-5,6,7,8-tetrahydroquinoline |
| b) 2-t-Butyl-5,6,7,8-tetrahydroquinoline | diisopropylcyanamide | 2-t-butyl-8-cyano-5,6,7,8-tetrahydroquinoline |
| c) 2-Methyl-5,6,7,8-tetrahydroquinoline | dicyclohexyl cyanamide | 8-cyano-2-methyl-5,6,7,8-tetrahydroquinoline |
| d) sym-Octahydroacridine | di-t-butylcyanamide | 4-cyano-sym-octahydroacridine |
| e) 3,4-Dimethyl-5,6,7,8-tetrahydroquinoline | diisopropyl cyanamide | 8-cyano-3,4-dimethyl-5,6,7,8-tetrahydroquinoline |
| f) 3,5-Dimethyl-5,6,7,8-tetrahydroquinoline | 1-cyanopiperidine | 8-cyano-3,5-dimethyl-5,6,7,8-tetrahydroquinoline |
| g) 3,6,-Dimethyl-5,6,7,8-tetrahydroquinoline | diethyl cyanamide | 8-cyano-3,6-dimethyl-5,6,7,8-tetrahydroquinoline |
| h) 1,2,3,4,7,8,9,10-Octahydrophenanthridine | diisopropyl cyanamide | 4-cyano-1,2,3,4,7,8,9,10-Octahydrophenanthridine |
| i) 2,3,5,6,7,8-Hexahydro-1-H-cyclopenta[b] quinoline | dimethyl cyanamide | 5-cyano-2,3,5,6,7,8-hexahydro-1-H-cyclopenta [b]quinoline |
| j) 3-Methyl-cyclopenteno[b] pyridine | N-t-butyl, N-cyclohexyl cyanamide | 7-cyano-3-methyl-cyclopenteno[b] pyridine |

Example 6

3-methyl-5,6,7,8-tetrahydroquinoline-8-thiocarboxamide

A) 8-Cyano-3-methyl-5,6,7,8-tetrahydroquinoline (8.8 g, 50 mM) in dichloromethane (50 ml) and diethyl dithiophosphate (8.4 ml, 50 mM) were treated with HCl gas at reflux. After 2.5 hours reaction mixture was cooled to ambient temperature and ether (250 ml) added. The resulting precipitate was removed by filtration, washed with ether and recrystallised from ethanol to give the title compound as the hydrochloride (11.1 g, 90%). mp 219°C identical with an authentic sample — see Example 18 of U.K. Patent 1432378.

B) The above reaction was repeated using dichloroethane as solvent instead of dichloromethane. The reaction was complete in 1 hour giving the title compound as the hydrochloride in 95% yield.

Example 7

4-methyl-5,6,7,8-tetrahydroquinoline-8-thiocarboxamide

A mixture of 8-cyano-4-methyl-5,6,7,8-tetrahydroquinoline (73 g, 0.35 M), diethyl dithiophosphate (60 ml, 0.36 M) and dichloroethane (350 ml) was treated with HCl gas at reflux over 4 hours. The mixture was then cooled to ambient temperature, filtered and the product washed with dichloroethane, then recrystallised from methanol — ether to give the title compound as the hydrochloride (74.5 g, 88%) mp 213°C identical with an authentic sample — see Example 1 of UK Patent 1463669.

Example 8

Following the procedure of Example 6A the nitriles listed in Example 5 are converted to the corresponding thioamides:

a) 2-Phenyl-5,6,7,8-tetrahydroquinoline-8-thiocarboxamide.
b) 2-t-butyl-5,6,7,8-tetrahydroquinoline-8-thiocarboxamide.
c) 2-methyl-5,6,7,8-tetrahydroquinoline-8-thiocarboxamide.
d) sym-octahydroacridine-4-thiocarboxamide.
e) 3,4-dimethyl-5,6,7,8-tetrahydroquinoline-8-thiocarboxamide.
f) 3,5-dimethyl-5,6,7,8-tetrahydroquinoline-8-thiocarboxamide.
g) 3,6-dimethyl-5,6,7,8-tetrahydroquinoline-8-thiocarboxamide.
h) 1,2,3,4,7,8,9,10-octahydrophenanthridine-4-thiocarboxamide.
i) 2,3,5,6,7,8-hexahydro-1-H-cyclopenta[b]quinoline-5-thiocarboxamide.
j) 3-methyl-cyclopenteno[b]pyridine-7-thiocarboxamide

Example 9

5,6,7,8-Tetrahydroquinoline-8-thiocarboxamide

A mixture of 8-cyano-5,6,7,8-tetrahydroquinoline (5.4 g, 34 mM) dichloromethane (100 ml) and diethyl dithiophosphate (5.75 ml) was maintained at reflux and treated with hydrogen chloride gas. After 8 hours the mixture was cooled to ambient temperature and diluted with ether. The resulting precipitate was washed well with ether and recrystallised from methanol/ether to give the title compound as the hydrochloride (7.0 g 90%) mp 263°C.

Example 10

8-Cyano-4-methyl-5,6,7,8-tetrahydroquinoline

A solution of 1.55 M butyl lithium in hexane (35.5 ml, 55 mM) maintained at 0° to 5°C. under argon was treated successively with N-t-butylcyclohexylamine (4.65 g, 30 mM) and a solution of 4-methyl-5,6,7,8-tetrahydroquinoline (3.67 g, 25 mM) in tetrahydrofuran (10 ml). After 0.5 hours at about 5°C a solution of di-isopropylcyanamide (3.5 g, 28 mM) was added in tetrahydrofuran (10 ml) and the mixture stirred a further 0.5 hours at 0°C. The mixture was quenched with water (50 ml), the aqueous layer extracted with toluene (50 ml), the combined organic phases dried and evaporated. Removal of t-butylcyclohexylamine under reduced pressure left an oil which contained (nmr analysis) 89% of the title compound and 11% 4-cyanomethyl-5,6,7,8-tetrahydroquinoline. Pure title compound was isolated by conversion to the hydrochloride in methanol as in Example 1.

Example 11

7-Cyanocyclopenteno[b]-pyridine

A solution of lithium diisopropylamide [made ex 1.55 M BuLi/hexane (64.5 ml, 0.1 M), diisopropylamine (14 ml, 0.1 M) tetrahydrofuran (30 ml)] maintained at 0°C. under argon was treated with a solution of cyclopenteno[b] pyridine (5.96 g, 50 mM) in tetrahydrofuran (10 ml). After 0.5 hours a solution of diisopropylcyanamide (7.0 g, 55 mM) in tetrahydrofuran (10 ml) was added. After a further 0.5 hours the reaction mixture was quenched with water, extracted with toluene, the organic phase washed with water, dried and evaporated. The residue was dissolved in ether/isopropanol and treated with an excess of ethereal hydrogen chloride. Removal of the resulting precipitate by filtration gave the title compound as the hydrochloride (5.6 g)mp 170°C.(sublimes)

Example 12

4-Cyano-1,2,3,4,5,6,7,8-octahydroacridine

A solution of lithium diisopropylamide [ex 1.55 M BuLi/hexane (32.3 ml, 50 mM), diisopropylamine (3.5 ml, 25 mM), tetrahydrofuran (20 ml)] maintained at 0°C. under an argon atmosphere was treated with a solution of octahydroacridine (4.68 g, 25 mM) in the tetrahydrofuran (10 ml). After 0.5 hours a solution of N-t-butyl-N-cyclohexylcyanamide (4.6 g, 28 mM) in tetrahydrofuran (10 ml) was added and the mixture stirred overnight. The reaction was quenched with water, extracted with toluene and the organic phase washed with water, dried and evaporated. The residue, dissolved in ethyl acetate, was passed through a short silica column to yield the title compound (3.8 g) ml. 88—90°C.

## Claims

1. A process for preparing compounds of Formula

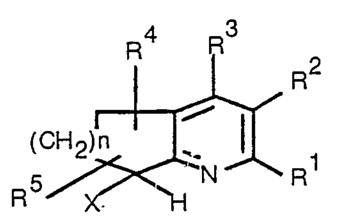

(I)

or acid addition salts thereof, wherein $R^1$, $R^2$, $R^3$, $R^4$ and $R^5$ are the same or different and represent hydrogen, alkyl, cycloalkyl, aralkyl, or aryl radicals, any of which radicals may be substituted or $R^1$ and $R^2$ taken together, or $R^2$ and $R^3$ taken together, form a 5, 6 or 7 membered ring which may be saturated or unsaturated and substituted or unsubstituted, $R^4$ and $R^5$ may also represent alkoxy, n is 1, 2 or 3 and X is CN or CSNHR wherein R is hydrogen or lower alkyl characterised in that a compound of Formula II

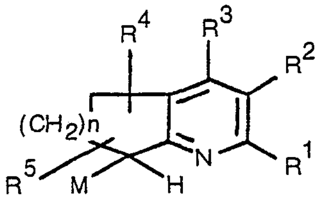

(II)

wherein $R^1$, $R^2$, $R^3$, $R^4$, $R^5$ and n are as defined above, and M is lithium, sodium, potassium, or MgHal, where Hal is chlorine, bromine or iodine, is reacted with a compound of formula RaRbNCN wherein Ra and Rb are the same or different and represent alkyl, cycloalkyl or aralkyl, or Ra and Rb may be joined to form a heterocyclic ring with the nitrogen and the product is treated with a proton source to obtain a nitrile of Formula I wherein X is CN and if desired the nitrile is treated with a sulphurising agent to obtain a thioamide of Formula I wherein X is CSNHR and optionally the compound of Formula I wherein X is CN or CSNHR is isolated as an acid addition salt.

2. A process as claimed in Claim 1, characterised in that the compound RaRbNCN is one in which Ra and Rb are chosen from secondary and tertiary alkyl radicals and cycloalkyl radicals.

3. A process as claimed in Claim 2, characterised in that the compound RaRbNCN is diisopropylcyanamide or N-t-butyl-N-cyclohexylcyanamide.

4. A process as claimed in any one of the preceding claims, characterised in that the reaction of Compound II and RaRbNCN is carried out by adding Compound II to a solution of RaRbNCN.

5. A process as claimed in any one of Claims 1—3, characterised in that the reaction of Compound II with RaRbNCN is carried out in the presence of a metal amide.

6. A process as claimed in Claim 5, characterised in that the metal amide has Formula MA wherein M is sodium, potassium, or lithium and A is a secondary amine radical $—NR^9R^{10}$ wherein $R^9$ and $R^{10}$ have the same meanings as Ra and Rb.

7. A process as claimed in Claim 6, characterised in that the compound RaRbNCN is diisopropylcyanamide and the secondary amine radical is N-t-butyl-N-cyclohexylamino.

8. A process as claimed in any one of the preceding claims, characterised in that the proton source is water (which may be in the form of an aqueous acid or other aqueous medium), an alkanol of 1—6 carbon atoms, or ammonium chloride.

9. A process as claimed in any one of the preceding claims, characterised in that a nitrile is prepared and treated with a dithiocompound of formula $(R^8)_2PSSH$ wherein $R^8$ is alkyl, aralkyl, aryl, alkoxy, aralkoxy, aryloxy, in the presence of a hydrogen halide to obtain a thioamide of Formula I wherein X is $CSNH_2$ and this is isolated as the free base or an acid addition salt thereof.

10. A process as claimed in any one of the preceding claims characterised in that the compound of Formula I which is prepared has the Formula (III)

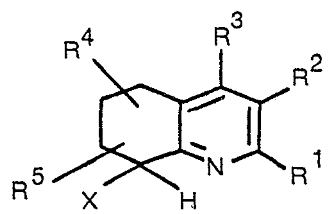

(III)

wherein $R^1$, $R^2$ and $R^3$ are the same or different and represent hydrogen or alkyl of 1 to 6 carbon atoms, $R^4$ and $R^5$ are the same or different and represent hydrogen, lower alkyl of 1 to 6 carbon atoms or lower alkoxy of 1 to 6 carbon atoms and X is CN or $CSNH_2$, in which a starting compound of Formula III wherein X is M and M is sodium potassium or lithium is reacted with the compound RaRbNCN, wherein Ra and Rb are as defined in Claim 1, and the product of Formula III is isolated as a free base or an acid addition salt.

11. A process as claimed in Claim 10, characterised in that the starting compound is one in which $R^1$, $R^2$, $R^4$ and $R^5$ are hydrogen and $R^3$ is lower alkyl.

12. A process as claimed in Claim 12, characterised in that the compound prepared is one in which $R^3$ is methyl.

13. A process as claimed in Claim 10, 11 or 12 characterised in that the starting compound of Formula III wherein X is M, is prepared by adding a corresponding compound where X is hydrogen to solution of a small excess of a metal alkyl $MR^6$ where $R^6$ is alkyl or aryl and M is sodium, potassium or

lithium or to a solution of up to 2.5 molar equivalents of a metal amide MA wherein M is sodium potassium or lithium and A is an amine radical.

**Revendications**

1. Procédé de préparation de composés de formule

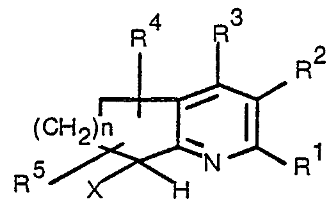

(I)

où $R^1$, $R^2$, $R^3$, $R^4$ et $R^5$ sont identiques ou différents et représentent des atomes d'hydrogène ou radicaux alcoyle, cyclo-alcoyle, aralcoyle ou aryle, chacun de ces radicaux pouvant être substitué, ou bien $R^1$ et $R^2$ pris ensemble ou $R^2$ et $R^3$ pris ensemble forment un cycle à 5, 6 ou 7 chaînons qui peut être saturé ou insaturé et substitué ou non substitué, $R^4$ et $R^5$ peuvent également représenter des radicaux alcoxy, n représente 1, 2 ou 3 et X représente CN ou CSNHR, où R représente un atome d'hydrogène ou radical alcoyle inferieur, ou de leurs sels d'addition d'acides, caractérisé en ce qu'un composé de formule II

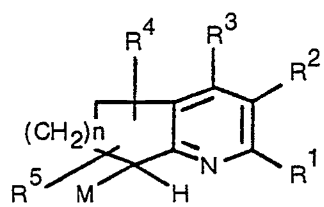

(II)

où $R^1$, $R^2$, $R^3$, $R^4$, $R^5$ et n sont tels que définis ci-dessus et M représente le lithium, le sodium, le potassium ou MgHal, où Hal représente le chlore, le brome ou l'iode, est mis à réagir avec un composé de formule RaRbNCN, où Ra et Rb sont identiques ou différents et représentent des radicaux alcoyle, cycloalcoyle ou aralcoyle, ou bien Ra et Rb peuvent être réunis pour former un hétérocycle avec l'atome d'azote auquel ils sont unis, et le produit est traité à l'aide d'une source de protons pour donner un nitrile de formule I où X représente CN et si la chose est souhaitée, le nitrile est traité à l'aide d'un agent de sulfuration pour donner un thioamide de formule I où X représente CSNHR et éventuellement le composé de formule I où X représente CN ou CSNHR est isolé sous forme de sel d'addition d'acide.

2. Procédé suivant la revendication 1, caractérisé en ce que le composé RaRbNCN est un composé dans lequel Ra et Rb sont choisis parmi les radicaux alcoyle secondaires et tertiaires et radicaux cycloalcoyle.

3. Procédé suivant la revendication 2, caractérisé en ce que le composé RaRbNCN est le diisopropylcyanamide ou le N-t-butyl-N-cyclohexylcyanamide.

4. Procédé suivant l'une quelconque des revendications précédentes, caractérisé en ce que la réaction du composé II et de RaRbNCN est exécutée par addition du composé II à une solution de RaRbNCN.

5. Procédé suivant l'une quelconque des revendications 1 à 3, caractérisé en ce que la réaction du composé II avec RaRbNCN est exécutée en présence d'un amidure métallique.

6. Procédé suivant la revendication 5, caractérisé en ce que l'amidure métallique est de formule MA où M représente le sodium, le potassium ou le lithium et A représente un radical d'amine secondaire $-NR^9R^{10}$, où $R^9$ et $R^{10}$ ont les mêmes significations que Ra et Rb.

7. Procédé suivant la revendication 6, caractérisé en ce que le composé RaRbNCN est le diisopropylcyanamide et le radical d'amine secondaire est le radical N-t-butyl-N-cyclohexylamino.

8. Procédé suivant l'une quelconque des revendications précédentes, caractérisé en ce que la source de protons est l'eau (qui peut se présenter sous forme d'un acide aqueux ou d'un autre milieu aqueux), un alcanol de 1 à 6 atomes de carbone ou le chlorure d'ammonium.

9. Procédé suivant l'une quelconque des revendications précédentes, caractérisé en ce qu'un nitrile est préparé et traité à l'aide d'un dithiocomposé de formule $(R^8)_2PSSH$ où $R^8$ représente un radical alcoyle, aralcoyle, aryle, alcoxy, aralcoxy ou aryloxy, en présence d'un halogénure d'hydrogène, pour donner un thio-amide de formule I où X représente $CSNH_2$ et celui-ci est isolé à l'état de base libre ou à l'état de sel d'addition d'acide.

10. Procédé suivant l'une quelconque des revendications précédentes, caractérisé en ce que le composé de formule I qui est préparé est de formule (III)

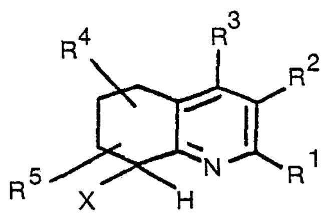

(III)

où $R^1$, $R^2$ et $R^3$ sont identiques ou différents et représentent des atomes d'hydrogène ou radicaux alcoyle de 1 à 6 atomes de carbone, $R^4$ et $R^5$ sont identiques ou différents et représentent des atomes d'hydrogène ou radicaux alcoyle inférieurs de 1 à 6 atomes de carbone ou alcoxy inférieurs de 1 à 6 atomes de carbone et X représente CN ou $CSNH_2$, suivant lequel un composé de départ de formule III où X représente M et M représente le sodium, le potassium ou le lithium, est mis à réagir avec le composé RaRbNCN, où Ra et Rb sont tels que définis dans la revendication 1, et le produit de formule III est isolé à l'état de base libre ou de sel d'addition d'acide.

11. Procédé suivant la revendication 10, caractérisé en ce que le composé de départ est un composé dans lequel $R^1$, $R^2$, $R^4$ et $R^5$ représentent des atomes d'hydrogène et $R^3$ représente un radical alcoyle inférieur.

12. Procédé suivant le revendication 11, caractérisé en ce que le composé préparé est un composé dans lequel $R^3$ représente un radical méthyle.

13. Procédé suivant la revendication 10, 11 ou 12, caractérisé en ce que le composé de départ de formule III où X représente M est préparé par addition d'un composé correspondant où X représente un atome d'hydrogène à une solution d'un petit excès d'un alcoyl-métal $MR^6$ où $R^6$ représente un radical alcoyle ou aryle et M représente le sodium, le potassium ou le lithium ou à une solution de jusqu'à 2,5, équivalents molaires d'un amidure métallique MA où M représente le sodium, le potassium ou le lithium et A représente un radical d'amine.

**Patentansprüche**

1. Verfahren zum Herstellen von Verbindungen der Formel

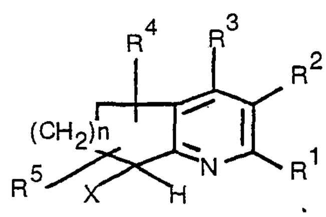

(I)

oder Säuereadditionssalzen hievon, worin $R^1$, $R^2$, $R^3$, $R^4$ und $R^5$ gleich oder verschieden sind und Wasserstoff, Alkyl, Cycloalkyl, Aralkyl oder Aryl bedeuten, wovon jedes substituiert sein kann, oder $R^1$ und $R^2$ miteinander genommen oder $R^2$ und $R^3$ miteinander genommen, einen 5-, 6- oder 7-gliedrigen Ring bilden, der gesättigt oder ungesättigt und substituiert oder unsubstituiert sein kann, $R^4$ und $R^5$ auch Alkoxy darstellen können, n 1, 2 oder 3 ist und X CN oder CSNHR bedeutet, wobei R Wasserstoff oder nied. Alkyl ist, dadurch gekennzeichnet, daß eine Verbindung der Formel (II)

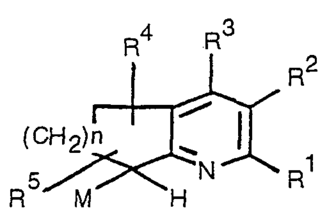

(II)

worin $R^1$, $R^2$, $R^3$, $R^4$ $R^5$ und n wie oben definiert sind und M Lithium, Natrium, Kalium oder MgHal bedeutet, wobei Hal Chlor, Brom oder Jod ist, mit einer Verbindung der Formel RaRbNCN, worin Ra und Rb gleich oder verschieden sind und Alkyl, Cycloalkyl oder Aralkyl bedeuten oder Ra und Rb unter Bildung eines heterocyclischen Ringes mit dem Stickstoff verbunden sein können, umgesetzt und das Produkt mit einer Protonenquelle behandelt wird, wobei ein Nitril der der Formel (I), worin X CN bedeutet, erhalten wird und, wenn gewünscht, das Nitril mit einem Sulfurierungsmittel behandelt wird, wobei ein Thioamid der Formel (I), worin X die Bedeutung CSHNR hat, erhalten wird und gegebenenfalls die Verbindung der Formel (I), worin X CN oder CSNHR ist, als Säureadditionssalz isoliert wird.

2. Verfahren wie in Anspruch 1 beansprucht, dadurch gekennzeichnet, daß die Verbindung RaRbNCN eine ist, worin Ra und Rb aus sek.-und tert.Alkylresten und Cycloalkylresten gewählt sind.

3. Verfahren wie in Anspruch 2 beansprucht, dadurch gekennzeichnet, daß die Verbindung RaRbNCN Diisopropylcyanamid oder N-tert.Butyl-N-cyclohexylcyanamid ist.

4. Verfahren wie in einem der vorhergehenden Ansprüche beansprucht, dadurch gekennzeichnet, daß die Reaktion der Verbindung (II) und RaRbNCN durch Zusetzen der Verbindung (II) zu einer Lösung von RaRbNCN durchgeführt wird.

5. Verfahren wie in einem der Ansprüche 1 bis 3 beansprucht, dadurch gekennzeichnet, daß die Reaktion der Verbindung (II) mit RaRbNCN in Anwesenheit eines Metallamids durchgeführt wird.

6. Verfahren wie in Anspruch 5 beansprucht, dadurch gekennzeichnet, daß das Metallamid die Formel MA aufweist, worin M Natrium, Kalium oder Lithium bedeutet und A ein sek.Aminrest —$NR^9R^{10}$ ist, wobei $R^9$ und $R^{10}$ die gleichen Bedeutungen wie Ra und Rb haben.

7. Verfahren wie in Anspruch 6 beansprucht, dadurch gekennzeichnet, daß die Verbindung RaRbNCN Diisopropylcyanamid ist und der sek.- Aminrest N-tert.Butyl-N-cyclohexylamino ist.

8. Verfahren wie in einem der vorhergehenden Ansprüche beansprucht, dadurch gekennzeichnet, daß die Protonenquelle Wasser (das in Form einer wässerigen Säure oder eines anderen wässerigen Mediums sein kann), ein Alkanol mit 1 bis 6 C-Atomen oder Ammoniumchlorid ist.

9. Verfahren wie in einem der vorhergehenden Ansprüche beansprucht, dadurch gekennzeichnet, daß ein Nitril hergestellt und mit einer Dithioverbindung der Formel $(R^8)_2PSSH$, worin $R^8$ Alkyl, Aralkyl, Aryl, Alkoxy, Aralkoxy oder Aryloxy ist, in Anwesenheit eines Halogenwasserstoffes behandelt wird, wobei ein Thioamid der Formel (I), worin X $CSNH_2$ bedeutet, erhalten wird und dieses als freie Base oder als Säureadditionssalz hievon isoliert wird.

10. Verfahren wie in einem der vorhergehenden Ansprüche beansprucht, dadurch gekennzeichnet, daß die Verbindung der Formel (I), die hergestellt wird, die Formel (III)

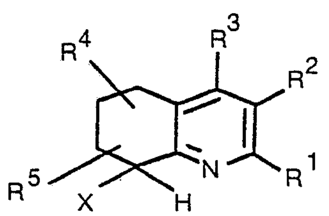

(III)

aufweist, worin $R^1$, $R^2$ und $R^3$ gleich oder verschieden sind und Wasserstoff oder Alkyl mit 1 bis 6 C-Atomen bedeuten, $R^4$ und $R^5$ gleich oder verschieden sind und Wasserstoff, nied.Alkyl mit 1 bis 6 C-Atomen oder nied.Alkoxy mit 1 bis 6 C-Atomen bedeuten und X CN oder $CSNH_2$ darstellt, wobei eine Ausgangsverbindung der Formel (III), worin X M ist und M Natrium, Kalium oder Lithium bedeutet, mit der Verbindung RaRbNCN, worin Ra und Rb wie in Anspruch 1 definiert sind, ungesetzt und das Produkt der Formel (III) als freie Base oder Säureadditionssalz isoliert wird.

11. Verfahren wie in Anspruch 10 beansprucht, dadurch gekennzeichnet, daß die Ausgangsverbindung eine ist, worin $R^1$, $R^2$, $R^4$ und $R^5$ Wasserstoff und $R^3$ nied.Alkyl sind.

12. Verfahren wie in Anspruch 12 beansprucht, dadurch gekennzeichnet, daß die hergestellte Verbindung eine ist, worin $R^3$ Methyl ist.

13. Verfahren wie in Anspruch 10, 11 oder 12 beansprucht, dadurch gekennzeichnet, daß die Ausgangsverbindung der Formel (III), worin X M bedeutet, durch Zusetzen einer entsprechenden Verbindung, worin X Wasserstoff ist, zu einer Lösung eines geringen Uberschusses eines Metallalkyls $MR^6$, wobei $R^6$ Alkyl oder Aryl bedeutet und M Natrium, Kalium oder Lithium ist, oder zu einer Lösung von bis zu 2,5 Moläquivalenten eines Metallamids MA, worin M Natrium, Kalium oder Lithium ist und A einen Aminrest darstellt, hergestellt wird.